# EUROPÄISCHE PATENTSCHRIFT

(11) **EP 1 017 278 B1**
(45) Veröffentlichungstag und Bekanntmachung des Hinweises auf die Patenterteilung: **12.11.2003**
(21) Anmeldenummer: 98951395.7
(22) Anmeldetag: 15.09.1998
(51) Int. Cl.: A01N 37/00

(54) **VERWENDUNG VON QUATERNÄREN CARBONSÄUREALKANOLAMINESTERSALZEN ALS MIKROBIZIDE WIRKSTOFFE**
USE OF QUATERNARY CARBOXYLIC ACID ALKANOLAMINE ESTER SALTS AS MICROBICIDE AGENTS
UTILISATION DE SELS QUATERNAIRES D'ALCANOLAMINESTER D'ACIDE CARBOXYLIQUE COMME AGENTS MICROBICIDES

(30) Priorität: 24.09.1997 DE 19742222
(43) Veröffentlichungstag der Anmeldung: 12.07.2000
(73) Patentinhaber: Cognis Deutschland GmbH & Co. KG, 40589 Düsseldorf (DE)
(72) Erfinder: STELTER, Norbert, D-40789 Monheim (DE); UPHUES, Günter, D-40789 Monheim (DE)
(86) Internationale Anmeldenummer: EP9805840
(87) Internationale Veröffentlichungsnummer: WO99015013

(56) Entgegenhaltungen:
- EP-A- 0 059 980
- EP-A- 0 186 052
- DE-A- 4 105 536
- NL-C- 1 001 114
- US-A- 4 824 867
- CHEMICAL ABSTRACTS, vol. 76, no. 12, 20. März 1972 Columbus, Ohio, US; abstract no. 61261, KOMKOV, I. P. ET AL: "Surface-active quaternary ammonium salts of o-acylcholines" XP002092702 & IZV. VYSSH. UCHEB. ZAVED., KHIM. KHIM. TEKHNOL. (1971), 14(9), 1369-73 CODEN: IVUKAR,
- CHEMICAL ABSTRACTS, vol. 106, no. 19, 1987 Columbus, Ohio, US; abstract no. 155889c, XP002092703 & CS 233 421 A (I. CSIBA ET AL) 15. August 1986
- CHEMICAL ABSTRACTS, vol. 120, no. 21, 1994 Columbus, Ohio, US; abstract no. 269666x, XP002092704 & CS 276 206 A (I. CSIBA ET AL) 15. April 1992

## Beschreibung

Die Erfindung betrifft die Verwendung von quaternären Carbonsäurealkanolaminestersalzen als mikrobizide Wirkstoffe, insbesondere in Wasch- und Reinigungsmitteln, Desinfektionsmitteln, Konservierungsmitteln, Weichspülern, Kosmetika, Kühlschmieremulsionen und in Anstrichmitteln. Des weiteren betrifft die Erfindung Wasch- und Reinigungsmittel, Weichspüler, Desinfektionsmittel, Kosmetika, Konservierungsmittel, Anstrichmittel und Kühlschmieremulsionen, die diese quaternären Carbonsäurealkanolaminestersalze enthalten.

Mikrobizide Wirkstoffe werden in der Regel eingesetzt, um Mikroorganismen an ihrer Vermehrung zu hindern oder ganz abzutöten. Bislang haben sich als mikrobizide Wirkstoffe quaternäre Ammoniumverbindungen wie Benzalkoniumchlorid oder Cetyltrimethylammoniumchlorid gut bewährt. Es hat sich jedoch gezeigt, daß derartige Verbindungen in der Kläranlage nur schwer biologisch abbaubar sind. Es besteht demnach ein Bedürfnis nach mikrobizid wirkenden Verbindungen, die in ihrer Leistungsfähigkeit mit den quaternären Ammoniumverbindungen vergleichbar sind, aber über eine deutlich verbesserte biologische Abbaubarkeit verfügen.

Aus der amerikanischen Patentschrift **US 3,910,971** sind quaternäre Carbonsäurealkanolaminestersalze als mikrobizide Wirkstoffe bekannt, die durch Umsetzung von 2-Dimethylamino-2-methyl-1-propanol mit Fettsäuren und anschließender Quaternierung hergestellt werden. Diese Verbindungen zeigen jedoch gegenüber dem gramnegativen Bakterium Pseudomonas aeruginosa kaum Wirksamkeit, was angesichts des häufig auftretenden Wasserkeimes, der insbesondere zu Produktverkeimungen führt, von Nachteil ist.

In der europäischen Patentanmeldung EP 0 461 419 werden Poly(oxyalkylen)aminoalkanolester sowie deren Ammoniumverbindungen beschrieben, die ebenfalls eine mikrobizide Wirkung aufweisen sollen. Diese Verbindungen weisen jedoch zwingend mindestens einen Hydroxyisopropylrest auf und tragen an dem Stickstoffatom eine Methylgruppe. Über die Wirksamkeit dieser Verbindungen gegenüber Bakterien, insbesondere gegenüber dem häufig vorkommenden Bakterium Pseudomonas aeruginosa, werden keine Angaben gemacht.

Aus der DE-A 41 05 536 sind biozide Mittel bekannt, die quaternierte Ammoniumverbindungen enthalten, wobei diese allerdings nur eine Estergruppe im Molekül enthalten.

Auch in der NL-A 1 001 114 werden biozide Mittel offenbart, die quaternierte Ammoniumverbindungen enthalten, wobei auch diese nur Monoesterverbindungen darstellen.

Aus der US-A 4,824,867 ist eine Methode zur Emulgierung von Bakterienwachstum unter Verwendung von quatemierten Ammoniumverbindungen bekannt, wobei auch in diesem Falle nur Monoesterverbindungen konkret offenbart werden.

Die EP-A 0 059 980 offenbart mikrobiozide Konservierungsmittel enthaltend Laurinsäureestersalze, wobei auch in diesem Fall der Stickstoff drei direkt gebundenen Methylgruppen enthält und somit konkret nur Monoesterverbindungen offenbart werden.

Aus der Offenbarung "Chemical Abstracts", Vol. 106, No. 19, 1987, Columbus, Ohio, Abstract no. 155889c und CS 233 421 A vom 15.08.1986 werden allgemein oberflächenaktive Verbindungen vom Typ der Esterquats offenbart, die exzellente bakterizide Eigenschaften aufweisen. Auch hier fehlt es an der Offenbarung von Di- oder Tri-esterverbindungen gemäß der vorliegenden Erfindung.

Aus der Offenbarung "Chemical Abstracts", Vol. 76, No. 12 vom 20.03.1972 ,Columbus, Ohio, Abstract no. 61261 KOMKOV. I. P. et. al.: "Surface-active quaternary Ammonium salts of o-acylcholines", XP 002092702 & IZV. VYSSH. UCHEB. ZAVED., KHIM. KHIM. TEKHNOL. (1971), 14(9), 1369-72 werden antimikrobiell wirksame N-[2-(10-undecenoyloxy)ethyl]-NNNalkyldimethylammoniumbromide offenbart, die wirksam sind, das Wachstum bestimmter Bakterien zu beeinflussen. Auch diese Offenbarung betrifft Monoesterverbindungen von Ammoniumsalzen.

Aufgabe der vorliegenden Erfindung war es, mikrobizid wirksame Verbindungen zur Verfügung zu stellen, die in ihrer mikrobiziden Wirksamkeit mit denen der herkömmlichen quatemären Ammoniumverbindungen vergleichbar sind. Vor allem sollten die Verbindungen auch mikrobizid wirksam sein gegenüber dem häufig auftretenden Bakterium Pseudomönas aeruginosa. Des weiteren sollten die Verbindungen besser biologisch abbaubar sein als die herkömmlichen quaternären Ammoniumverbindungen.

Es wurde gefunden, daß spezielle quaternäre Carbonsäurealkanolaminestersalze diese Aufgabe erfüllen.

Ein Gegenstand der vorliegenden Erfindung betrifft daher die Verwendung von quaternären Carbonsäurealkanolaminestersalzen der allgemeinen Formel **(I)**, 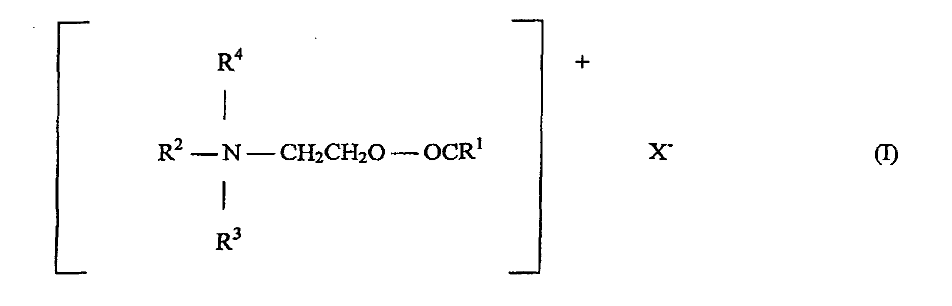 in der
R¹CO fiir einen Acylrest mit 2 bis 18 C-Atomen,
R² für einen Rest der Formel CH₂ - CH₂ - O - OCR¹,
R³ fiir einen Alkylrest mit 1 bis 16 C-Atomen oder für einen Rest der Formel CH₂ - CH₂ - O - R⁵
R⁴ für einen Alkylrest mit 1 bis 4 C-Atomen,
R⁵ für Wasserstoff und/oder R¹CO und
X⁻ für ein Anion steht
als mikrobizide Wirkstoffe.

Die quaternären Carbonsäurealkanolaminestersalze sind literaturbekannte Verbindungen, die häufig auch als "Esterquats" bezeichnet werden. In der Regel werden sie durch Veresterung der Alkanolamine mit Carbonsäuren in Gegenwart von unterphosphoriger Säure, Luftdurchleitung und nachfolgender Quaternierung hergestellt. Stellvertretend für den umfangreichen Stand der Technik sei an dieser Stelle auf die Druckschriften **US 3, 915,867, US 4,370,272, EP-A- 0 239 910, EP-A- 293 955 , EP-A- 295 739** und **EP -A- 309 052** verwiesen.

Im Sinne der Erfindung werden die quaternären Carbonsäurealkanolaminestersalze nach den an und für sich bekannten Methoden hergestellt, wobei zunächst Alkanolamine der Formel **(II**), 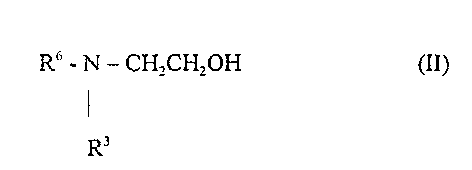 in der R⁶ und R³ unabhängig voneinander für einen Alkylrest mit 1 bis 16 C-Atomen oder für einen Rest der Formel CH₂ - CH₂ - O - R⁵ stehen,wobei im Falle, daß R⁶ und/oder R³ für den Rest der Formel CH₂CH₂OR⁵ steht, R⁵ = Wasserstoff bedeutet, mit Carbonsäuren der Formel R¹COOH verestert werden. Es ist auch möglich, die Alkanolamine der Formel (II) mit Estern der beschriebenen Carbonsäuren umzuestern und somit zu den erfindungsgemäß verwendeten Verbindungen der Formel (I) zu gelangen. Als Alkanolamine kommen Dialkylethanolamine (R⁶, R³= Alkylrest mit 1 bis 16 Kohlenstoffatomen) wie Dimethylethanolamin, Methylethylethanolamin, Diethylethanolamin, Methylbutylethanolamin und/oder Methylhexylethanolamin, Monoalkyldiethanolamine (R⁶= CH₂CH₂OH; R³= Alkylrest mit 1 bis 16 Kohlenstoffatome) wie Methyldiethanolamin, Ethyldiethanolamin und/oder Butyldiethanolamin, und/oder Triethanolamine (R⁶, R³= CH₂CH₂OH) in Betracht. Bevorzugt werden Monoalkyldiethanolamine und /oder Triethanolamine gewählt.

Als Carbonsäuren der Formel R¹COOH eignen sich aliphatische gesättigte und/oder ungesättigte Carbonsäuren, insbesondere aliphatische gesättigte Carbonsäuren wie Essigsäure, Propionsäure, Buttersäure, Capronsäure, Caprylsäure, Caprinsäure, Pelargonsäure, Laurinsäure, Myristinsäure, Palmitinsäure sowie deren technische Mischungen, wie sie beispielsweise bei der Druckspaltung natürlicher Fette und Öle anfallen. Bevorzugt werden aliphatische gesättigte Carbonsäuren mit 8 bis 12 Kohlenstoffatomen, so daß bevorzugt in Formel (I) R¹CO für einen aliphatischen gesättigten Acylrest mit 8 bis 12 Kohlenstoffatomen steht.

Das Einsatzverhältnis von Carbonsäuren bzw. Carbonsäureestern zu den Alkanolaminen wird durch den gewünschten Veresterungsgrad der freien Hydroxylgruppen der Alkanolamine bestimmt. Bei den bevorzugten Monoalkyldiethanolaminen und Triethanolamin können alle oder nur einige der freien Hydroxylgruppen mit den Carbonsäuren verestert werden. Sofern keine Veresterung der Hydroxylgruppe erfolgt, steht in der allgemeinen Formel (I) R⁵ für ein Wasserstoff. Sofern eine Veresterung erfolgt, steht in der allgemeinen Formel (I) R⁵ für R¹CO. Die Veresterung kann vollständig oder teilweise erfolgen. Bei einer teilweisen Veresterung liegt der durchschnittliche Veresterungsgrad der insgesamt freien Hydroxylgruppen bei den Monoalkyldiethanolaminen vorzugsweise im Bereich von 1,2 bis 1,7 und bei den Triethanolaminen vorzugsweise im Bereich von 1,2 bis 2,5, d.h. es liegen Mischungen von Mono-, Di- und ggf. Triestern der Di- bzw. Triethanolamine mit Carbonsäuren vor. Bevorzugt im Sinne der Erfindung werden mindestens zwei der freien Hydroxylgruppen bei dem Triethanolamin mit Carbonsäuren verestert. Anstelle der Carbonsäuren können auch deren Esterderivate mit den Alkanolaminen umsetzt werden.

Die anschließende Quatemierung wird nach den auf diesem Gebiet bekannten Verfahren durchgeführt und führt zu den quaternären Carbonsäurealkanolaminestersalzen der allgemeinen Formel (I). Zur Quaternierung werden Verbindungen der Formel R⁴X eingesetzt, wobei R⁴ die in Formel (I) gegebene Bedeutung aufweist, vorzugsweise steht R⁴ für eine Methylgruppe. Sofern X in der allgemeinen Formel (I) fiir Formiat, Acetat, Tartrat, Dicarboxylat, Citrat, Halogenid, Sulfat oder Nitrat steht, erfolgte nach der üblichen Quaternierung ein Anionenaustausch.

Besonders bevorzugt im Sinne der Erfindung werden Verbindungen der Formel (I) verwendet, in denen R² für einen Rest der Formel CH₂CH₂OR⁵ und R³ für eine Methylgruppe steht und/oder in denen R² und R³ für einen Rest der Formel CH₂CH₂OR⁵ steht, wobei R⁵ die angegebene Bedeutung hat. Beispiele für besonders geeignete Verbindungen sind Dimethyldiethanolammoniumdicaprylsäureester Methosulfat, Methyltriethanolammoniumtricaprylsäureester Methosulfat, Methyltriethanolammoniumdicaprylsäureester Methosulfat und Dimethyldiethanolammoniumdipelargonsäureester Methosulfat.

Die beschriebenen Verbindungen werden erfindungsgemäß als mikrobizide Wirkstoffe verwendet. Im Sinne der Erfindung werden unter dem Begriff der mikrobiziden Wirkstoffe solche Wirkstoffe verstanden, die in der Lage sind, Bakterien, Hefen, Schimmelpilze, Viren und Protisten abzutöten oder an ihrer Vermehrung zu hindern.

Derartige mikrobizide Wirkstoffe können in allen Produkten eingesetzt werden, in denen eine entsprechende Wirkung gewünscht wird. Vorzugsweise werden sie eingesetzt in Wasch- und Reinigungsmitteln, beispielsweise in Handgeschirrspülmittel, Allzweckreiniger, Sanitärreiniger, Weichspüler für Textilien zur antimikrobiellen Textilausrüstung, in Desinfektionsmitteln, beispielsweise im Haushalt und in Krankenhäusern, in Konservierungsmitteln, beispielsweise zur Konservierung von technischen Rohstoffen und Produkten, in Kühlschmieremulsionen, in Kosmetika und in Anstrichmitteln wie Leime, Farben und Holzschutzmitteln.

Die erfindungsgemäß verwendeten Verbindungen sind gegenüber einigen Bakterien schon wirksam in Mengen über 5 mg/l - berechnet als Aktivsubstanz und bezogen auf das Mittel. In der Regel beträgt die Einsatzmenge 5 bis 5000 mg/l, vorzugsweise 50 bis 1000 mg/l - berechnet als Aktivsubstanz und bezogen auf das Mittel. Bei konzentrierten Produkten können die Einsatzmengen der quatemären Carbonsäurealkanolaminestersalze auch darüber liegen. Werden die konzentrierten Produkte jedoch vom Endverbraucher mit Wasser verdünnt, liegen die Einsatzmengen wieder im oben angegebenen Bereich.

Die erfindungsgemäß verwendeten Verbindungen können pur oder in Form ihrer Lösungen zur Anwendung kommen. Als geeignete Lösungsmittel sind Wasser und/oder niedere Alkohole zu nennen, insbesondere Ethanol, Propandiol und/oder Isopropanol. Niedere Alkohole oder deren wäßrige Mischungen empfehlen sich vor allem für quaternäre Carbonsäurealkanolaminestersalze der beschriebenen Art, die mehr als zwei Acylreste mit mehr als 6 Kohlenstoffatomen enthalten.

Weitere Gegenstände der vorliegenden Erfindung betreffen Wasch- und Reinigungsmittel, die als mikrobizide Wirkstoffe quaternäre Carbonsäurealkanolaminestersalze enthalten, sowie Konservierungsmittel, die als mikrobizide Wirkstoffe quaternäre Carbonsäurealkanolaminestersalze enthalten, sowie Kosmetika, die als mikrobizide Wirkstoffe quaternäre Carbonsäurealkanolaminestersalze enthalten sowie Desinfektionsmittel, die quaternäre Carbonsäurealkanolaminestersalze enthalten, sowie Kühlschmieremulsionen, die quaternäre Carbonsäurealkanolaminestersalze enthalten und Weichspüler, die als mikrobizide Wirkstoffe quaternäre Carbonsäurealkanolaminestersalze enthalten. In den genannten Mitteln können die quaternären Carbonsäurealkanolaminestersalze in den oben angegebenen Mengen enthalten sein. Des weiteren können die Mittel natürlich andere mikrobizide Wirkstoffe und übliche weitere Bestandteile in üblichen Mengen enthalten.

So können in den Wasch- und Reinigungsmitteln anionische, nichtionische und/oder amphotere bzw. zwitterionische Tenside enthalten sein, die im folgenden exemplarisch genannt werden:

Anionische Tenside können sein: Alkylbenzolsulfonate, Alkansulfonate, Olefinsulfonate, Alkylethersulfonate, Glycerinethersulfonate, -Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Sulfosuccinate, Sulfosuccinamate, Sulfotriglyceride, Ethercarbonsäuren, Alkyloligoglucosidsulfate, Alkyl(ether)phosphate und Eiweißfettsäurekondensate. Nichtionische Tenside können sein: Fettalkoholpolyglycolether, Alkylphenolpolyglycolether, Fettaminpolyglycolether, Fettsäureamidpolyglycolether, Fettsäurepolyglycolester, alkoxylierte Triglyceride, Alkyloligoglykoside, Zuckerester, Sorbitanester, Polysorbate, Polyolfettsäureester, Aminoxide, Fettsäurealkanolamide, Alkyllactame und Fettsäure-N-alkylglucamide. Amphotere bzw. zwitterionische Tenside können sein: Alkylamidobetaine, Aminopropionate, Aminoglycinate, Imidazoliniumbetaine und Sulfobetaine.

Desweiteren können in den Wasch- und Reinigungsmitteln übliche Hilfsstoffe wie Builder, Salze, Bleichmittel, optische Aufheller, Vergrauungsinhibitoren, Lösungsvermittler und Enzyme enthalten sein.

Die Konservierungsmittel können als weitere Komponenten Alkohole, Aldehyde, Säuren, Ester, Phenole, Terpene und/oder Komplexbildner enthalten.

Die Desinfektionsmittel können ebenfalls eines oder mehrere der oben genannten anionischen, nichtionischen und/oder amphoteren bzw. zwitterionischen Tenside enthalten sowie Wasser, Alkohole, Komplexbildner und weitere mikrobizid wirkende Verbindungen.

Die Kühlschmieremulsionen enthalten in der Regel eine wäßrige Phase und eine organische Phase, wobei die organischen Phase meist von Mineralölen oder pflanzlichen Ölen gebildet wird. Des weiteren sind meist Emulgatoren, ggf. Extrem-pressure-Additive, Korrosionsinhibitoren und/oder Komplexbildner zugegen.

Die Kosmetika können ebenfalls eines oder mehrere der oben genannten anionischen, nichtionischen und/oder amphoteren bzw. zwitterionischen Tenside enthalten sowie ggf. Wasser bzw. eine Wasserphase, Alkohole, ggf. eine Ölphase, Emulgatoren sowie ggf. weitere mikrobizid wirkende Verbindungen. Des weiteren können übliche Additive wie Abraisivstoffe, Perlglanzpigmente, Farbstoffe, Duftstoffe, ggf. weitere Konservierungsmittel, Gelbildner und/oder konditionierende und pflegende Wirkstoffe enthalten sein.

### Beispiele

### A. Mikrobizide Wirksamkeit

Die mikrobizide Wirksamkeit der erfindungsgemäß verwendeten quatemären Carbonsäurealkanolaminestersalze wurde gegenüber folgenden ausgewählten Testkeimen bestimmt:
a) Staphylococcus aureus ATCC 6538:
b) Pseudomonas aruginosa ATCC 15442
c) Candida albicans ATCC 10231

Die Wirksamkeit der zu untersuchenden Verbindung wurde mit Hilfe des Suspensionstests in Anlehnung an die Richtlinien der Deutschen Gesellschaft für Hygiene und Mikrobiologie (DGHM) ermittelt. Unter Verwendung von Wasser einer Härte von 17°dH sowie ggf. von Isopropanol wurden Testlösungen hergestellt, die die in den Tabellen angegebenen Mengen an quaternären Carbonsäurealkanolaminestersalzen von 50, 100, 200, 400 und 800 mg/l enthielten.

Bei Raumtemperatur wurden jeweils 0,1 ml Testkeimsuspension in Reagenzgläser mit jeweils 10 ml der oben beschriebenen Testlösungen pipettiert und vermischt. Nach unterschiedlichen Einwirkzeiten von 5, 15, 30 und 60 Minuten wurden den Reagenzgläsern mit Hilfe einer Pipette 1 ml Material entnommen, und in 9 ml Inaktivatorlösung (Inaktivatorsubstanzen: 3,0 % Tween, 0,3 % Lecithin, 0,1 % Histidin) übertragen. Nach mindestens 5 und längstens 30 Minuten Inaktivierung erfolgte die Bestimmung der Lebendkeimzahl durch Ausplattierung auf Caseinpepton-Sojabohnenmehlpepton(CaSo)-Agarplatten. Unter Verwendung eines Spiralplaters wurde direkt der Inaktivierungsansatz auf CaSo-Agar ausplattiert. Die Proben wurden 24 bis 72 Stunden bei 37°C bebrütet und anschließend makroskopisch auf Wachstum beurteilt und auf diesem Weg die Abtötungszeit oder der Restkeimgehalt ermittelt. Dabei wird die Keimzahl einer unbelasteten Wasserkontrolle in das Verhältnis zur Keimzahl der inaktivierten Testlösung unter Berücksichtigung der Verdünnung gestellt. In der nachfolgenden Tabelle 1 sind die logarithmischen Reduktionsfaktoren angegeben, d.h. die Zehnerpotenzreduktion. Dabei gilt, je höher der Wert ist, desto besser ist die mikrobizide Wirkung. Die Angabe < bedeutet, daß der logarithmische Reduktionsfaktor unter dem angegebenen Wert bzw. bei der Angabe > über dem angegebenen Wert liegt. Die Abkürzung AKW steht für Anwendungskonzentration.

Getestet wurden im einzelnen die Verbindungen:
- Bsp. 1: Dimethyldiethanolammoniumdicaprylsäureester Methosulfat
- Bsp. 2: Methyltriethanolammoniumtricaprylsäureester Methosulfat
- Bsp. 3: Dimethyldiethanolammoniumdipelargonsäureester Methosulfat
- Bsp. 4: Trimethylethanolammoniumkokosfettsäureester Methosulfat
- Bsp. 5: Methyltriethanolammonium-di-laurinsäureester Methosulfat
- Bsp. 6: Methyltriethanolammonium-di-caprinsäureester Methosulfat
- Bsp. 7: Methyltriethanolammonium-di-caprylsäureester Methosulfat
- Bsp. 8: Dimethyldiethanolammonium-di-laurinsäureester Methosulfat
- Bsp. 9: Methyltriethanotammonium-tri-caprinsäureester Methosulfat
- Bsp. 10: Dimethyldiethanolammonium-di-caprinsäureester Methosulfat

**Tabelle 1**

| **Beispiel 1: Dimethyldiethanolammoniumdicaprylsäureester Methosulfat** | | | | | |
|---|---|---|---|---|---|
| **Staphylococcus aureus ATCC 6538** | | | | | |
| **Keimzahl 2,73 · 10**^{**7**} **/ ml** | | | | | |
| **AWK [mg/l]** | **50** | **100** | **200** | **400** | **800** |
| **5 Min** | < 1,32 | < 1,32 | < 1,32 | < 1,32 | < 1,32 |
| **15 Min** | < 1,71 | < 1,71 | < 1,71 | < 1,71 | < 3,59 |
| **30 Min** | < 1,23 | < 1,23 | < 1,23 | 1,95 | < 3,96 |
| **60 Min** | < 1,32 | < 1,32 | < 1,32 | 3,54 | > 5,32 |

| **Pseudomonas aeruginosa ATCC 15442** | | | | | |
|---|---|---|---|---|---|
| **Keimzahl 4,23 · 10**^{**7**} **/ ml** | | | | | |
| **AWK [mg/l]** | **50** | **100** | **200** | **400** | **800** |
| **5 Min** | < 1,83 | < 1,83 | < 1,83 | < 1,83 | > 5,83 |
| **15 Min** | < 1,47 | < 1,47 | < 1,47 | < 1,47 | > 5,47 |
| **30 Min** | < 1,55 | < 1,55 | < 1,55 | 3,22 | > 5,55 |
| **60 Min** | < 1,56 | < 1,56 | < 1,56 | 4,41 | > 5,56 |

| **Candida albicans ATCC 10231** | | | | | |
|---|---|---|---|---|---|
| **Keimzahl 2,59 · 10**^{**6**} **/ ml** | | | | | |
| **AWK [mg/l]** | **50** | **100** | **200** | **400** | **800** |
| **5 Min** | < 0,50 | < 0,50 | 1,53 | > 4,5 | > 4,50 |
| **15 Min** | < 0,37 | 0,41 | 2,53 | > 4,37 | > 4,37 |
| **30 Min** | < 0,35 | 0,60 | 2,91 | > 4,35 | > 4,35 |
| **60 Min** | < 0,36 | 0,70 | 3,61 | > 4,36 | > 4,36 |
| | | | | | |

| **Beispiel 2: Methyltriethanolammoniumtricaprylsäureester Methosulfat** | | | | | |
|---|---|---|---|---|---|
| **Staphylococcus aureus ATCC 6538** | | | | | |
| **Keimzahl 1,44 · 10**^{**7**} **/ ml** | | | | | |
| **AWK [mg/l]** | **50** | **100** | **200** | **400** | **800** |
| **5 Min** | 3,21 | 3,54 | 4,32 | > 5,56 | > 5,56 |
| **15 Min** | > 5,38 | > 5,38 | > 5,38 | > 5,38 | > 5,38 |
| **30 Min** | > 5,50 | > 5,50 | > 5,50 | > 5,50 | > 5,50 |
| **60 Min** | > 5,41 | > 5,41 | > 5,41 | > 5,41 | > 5,41 |

| **Pseudomonas aeruginosa ATCC 15442** | | | | | |
|---|---|---|---|---|---|
| **Keimzahl 6,27 · 10**^{**7**} **/ ml** | | | | | |
| **AWK [mg/l]** | **50** | **100** | **200** | **400** | **800** |
| **5 Min** | < 1,38 | < 1,38 | 1,38 | 4,08 | 4,42 |
| **15 Min** | < 1,70 | 3,25 | 3,95 | > 5,70 | > 5,70 |
| **30 Min** | 3,31 | 3,75 | 4,40 | > 5,68 | > 5,68 |
| **60 Min** | 3,98 | 4,49 | > 5,59 | > 5,59 | > 5,59 |

| **Candida albicans ATCC 10231** | | | | | |
|---|---|---|---|---|---|
| **Keimzahl 6,91 · 10**^{**6**} **/ ml** | | | | | |
| **AWK [mg/l]** | **50** | **100** | **200** | **400** | **800** |
| **5 Min** | 3,44 | > 4,73 | > 4,73 | > 4,73 | > 4,73 |
| **15 Min** | > 4,71 | > 4,71 | > 4,71 | > 4,71 | > 4,71 |
| **30 Min** | > 4,82 | > 4,82 | > 4,82 | > 4,82 | > 4,82 |
| **60 Min** | > 4,68 | > 4,68 | > 4,68 | > 4,68 | > 4,68 |
| | | | | | |

| **Beispiel 3: Dimethyldiethanolammoniumdipelargonsäureester Methosulfat** | | | | | |
|---|---|---|---|---|---|
| **Staphylococcus aureus ATCC 6538** | | | | | |
| **Keimzahl 2,41 · 10**^{**7**}**/ ml** | | | | | |
| **AWK [mg/l]** | **50** | **100** | **200** | **400** | **800** |
| **5 Min** | < 1,31 | < 1,31 | 2,05 | 3,25 | 3,78 |
| **15 Min** | 2,31 | 3,67 | > 5,74 | > 5,74 | > 5,74 |
| **30 Min** | 3.01 | > 5,19 | > 5,19 | > 5,19 | > 5,19 |
| **60 Min** | 4,85 | > 5,55 | > 5,55 | > 5,55 | > 5,55 |

| **Pseudomonas aeruginosa ATCC 15442** | | | | | |
|---|---|---|---|---|---|
| **Keimzahl 5,09 · 10**^{**7**} **/ ml** | | | | | |
| **AWK [mg/l]** | **50** | **100** | **200** | **400** | **800** |
| **5 Min** | < 1,59 | 4,36 | > 5,59 | > 5,59 | > 5,59 |
| **15 Min** | 2,99 | > 5,58 | > 5,58 | > 5,58 | > 5,58 |
| **30 Min** | 3,58 | > 5,67 | > 5,67 | > 5,67 | > 5,67 |
| **60 Min** | 3,77 | > 5,53 | > 5,53 | > 5,53 | > 5,53 |

| **Candida albicans ATCC 10231** | | | | | |
|---|---|---|---|---|---|
| **Keimzahl 4,82 · 10**^{**6**} **/ ml** | | | | | |
| **AWK [mg/l]** | **50** | **100** | **200** | **400** | **800** |
| **5 Min** | 2,86 | > 4,63 | > 4,63 | > 4,63 | > 4,63 |
| **15 Min** | > 4,63 | > 4,63 | > 4,63 | > 4,63 | > 4,63 |
| **30 Min** | > 4,66 | > 4,66 | > 4,66 | > 4,66 | > 4,66 |
| **60 Min** | > 4,67 | > 4,67 | > 4,67 | > 4,67 | > 4,67 |
| | | | | | |

| **Beispiel 4: Trimethylethanolammoniumcocosfettsäureester Methosulfat** | | | | | |
|---|---|---|---|---|---|
| 75%ig in Isopropanol | | | | | |
| **Staphylococcus aureus ATCC 6538** | | | | | |
| **Keimzahl 2,05 · 10**^{**7**} **/ ml** | | | | | |
| **AWK [mg/l]** | **50** | **100** | **200** | **400** | **800** |
| **5 Min** | <1,51 | <1,51 | <1,51 | 2,33 | 3,12 |
| **15 Min** | <1,36 | 1,78 | 3,4 | 4,14 | >5,36 |
| **30 Min** | < 1,38 | 3,03 | 4,38 | > 5,38 | > 5,38 |
| **60 Min** | 1,94 | 4,02 | > 5,26 | > 5,26 | > 5,26 |

| **Pseudomonas aeruginosa ATCC 15442** | | | | | |
|---|---|---|---|---|---|
| **Keimzahl 5,77 · 10**^{**7**} **/ ml** | | | | | |
| **AWK [mg/l]** | **50** | **100** | **200** | **400** | **800** |
| **5 Min** | < 1,51 | < 1,51 | < 1,51 | < 1,51 | 4,02 |
| **15 Min** | < 1,66 | < 1,66 | < 1,66 | 3,9 | > 5,66 |
| **30 Min** | < 1,46 | < 1,46 | < 1,46 | > 5,66 | > 5,66 |
| **60 Min** | < 1,47 | < 1,47 | < 3,35 | > 5,47 | > 5,47 |

| **Candida albicans ATCC 10231** | | | | | |
|---|---|---|---|---|---|
| **Keimzahl 7,05 · 10**^{**7**} **/ ml** | | | | | |
| **AWK [mg/l]** | **50** | **100** | **200** | **400** | **800** |
| **5 Min** | < 0,80 | 1,19 | 3,43 | > 4,80 | > 4,80 |
| **15 Min** | < 0,79 | 3,01 | > 4,79 | > 4,79 | > 4,79 |
| **30 Min** | 0,91 | > 4,80 | > 4,80 | > 4,80 | > 4,80 |
| **60 Min** | 1,40 | > 5,05 | > 5,05 | > 5,05 | > 5,05 |
| | | | | | |

| **Beispiel 5: Methyltriethanolammonium-di-laurinsäureester Methosulfat** | | | | | |
|---|---|---|---|---|---|
| **Staphylococcus aureus ATCC 6538** | | | | | |
| **Keimzahl 5,95 x 10**^{**9**} **/ ml** | | | | | |
| **AWK [mg/l]** | **50** | **100** | **200** | **400** | **800** |
| **5 Min** | < 1,79 | < 1,79 | 2,52 | 2,70 | 3,85 |
| **15 Min** | < 1,73 | 2,38 | 4,25 | > 5,73 | > 5,73 |
| **30 Min** | 2,53 | 3,80 | > 5,75 | > 5,75 | > 5,75 |
| **60 Min** | 3,88 | 5,21 | > 5,86 | > 5,86 | > 5,86 |

| **Pseudomonas aruginosa ATCC 15442** | | | | | |
|---|---|---|---|---|---|
| **Keimzahl 7,86 x 10**^{**9**} **/ ml** | | | | | |
| **AWK [mg/l]** | **50** | **100** | **200** | **400** | **800** |
| **5 Min** | < 1,85 | < 1,85 | < 1,85 | < 1,85 | < 1,85 |
| **15 Min** | < 1,79 | < 1,79 | < 1,79 | < 1,79 | 3,43 |
| **30 Min** | < 1,71 | < 1,71 | < 1,71 | < 1,71 | 3,95 |
| **60 Min** | < 1,81 | < 1,81 | < 1,81 | 3,84 | 4,40 |
| | | | | | |

| **Beispiel 6: Methyltriethanolammonium-di-caprinsäureester Methosulfat** | | | | | |
|---|---|---|---|---|---|
| **Staphylococcus aureus ATCC 6538** | | | | | |
| **Keimzahl 5,95 x 10**^{**9**} **/ ml** | | | | | |
| **AWK [mg/l]** | **50** | **100** | **200** | **400** | **800** |
| **5 Min** | 4,12 | 4,58 | 4,55 | 4,48 | 5,44 |
| **15 Min** | > 5,73 | > 5,73 | > 5,73 | > 5,73 | > 5,73 |
| **30 Min** | > 5,75 | > 5,75 | > 5,75 | > 5,75 | > 5,75 |
| **60 Min** | > 5,86 | > 5,86 | > 5,86 | > 5,86 | > 5,86 |

| **Pseudomonas aruginosa ATCC 15442** | | | | | |
|---|---|---|---|---|---|
| **Keimzahl 7,86 x 10**^{**9**} **/ ml** | | | | | |
| **AWK[mg/l]** | **50** | **100** | **200** | **400** | **800** |
| **5 Min** | < 1,85 | < 1,85 | < 1,85 | < 1,85 | < 1,85 |
| **15 Min** | < 1,79 | < 1,79 | < 1,79 | < 1,79 | 4,14 |
| **30 Min** | < 1,71 | < 1,71 | 3,35 | 4,18 | 4,76 |
| **60 Min** | < 1,81 | 3,19 | 3,89 | > 5,81 | > 5,81 |

| **Candida albicans ATCC 10231** | | | | | |
|---|---|---|---|---|---|
| **Keimzahl 8,95 x 10**^{**8**} **/ ml** | | | | | |
| **AWK [mg/l]** | **50** | **100** | **200** | **400** | **800** |
| **5 Min** | < 0,87 | 1,27 | 1,24 | 1,38 | 1,33 |
| **15 Min** | 1,64 | 3,03 | 3,66 | 3,51 | > 4,87 |
| **30 Min** | 1,93 | 3,75 | > 4,78 | > 4,78 | > 4,78 |
| **60 Min** | 2,62 | 4,15 | > 4,67 | > 4,67 | > 4,67 |
| | | | | | |

| **Beispiel 7: Methyltriethanolammonium-di-caprylsäureester Methosulfat** | | | | | |
|---|---|---|---|---|---|
| **Staphylococcus aureus ATCC 6538** | | | | | |
| **Keimzahl 3,5 x 10**^{**9**} **/ ml** | | | | | |
| **AWK [mg/l]** | **50** | **100** | **200** | **400** | **800** |
| **5 Min** | > 5,75 | > 5,75 | > 5,75 | > 5,75 | > 5,75 |
| **15 Min** | > 5,53 | > 5,53 | > 5,53 | > 5,53 | > 5,53 |
| **30 Min** | > 5,42 | > 5,42 | > 5,42 | > 5,42 | > 5,42 |
| **60 Min** | > 5,69 | > 5,69 | > 5,69 | > 5,69 | > 5,69 |

| **Pseudomonas aruginosa ATCC 15442** | | | | | |
|---|---|---|---|---|---|
| **Keimzahl 9,14 x 10**^{**9**} **/ ml** | | | | | |
| **AWK [mg/l]** | **50** | **100** | **200** | **400** | **800** |
| **5 Min** | < 1,77 | 3,64 | 4,59 | 5,32 | > 5,77 |
| **15 Min** | < 1,72 | > 5,72 | > 5,72 | > 5,72 | > 5,72 |
| **30 Min** | < 1,76 | > 5,76 | 5,76 | > 5,76 | > 5,76 |
| **60 Min** | 3,65 | > 5,86 | > 5,86 | > 5,86 | > 5,86 |

| **Candida albicans ATCC 10231** | | | | | |
|---|---|---|---|---|---|
| **Keimzahl 1,81 x 10**^{**8**} **/ ml** | | | | | |
| **AWK [mg/l]** | **50** | **100** | **200** | **400** | **800** |
| **5 Min** | > 4,31 | > 4,31 | > 4,31 | > 4,31 | > 4,31 |
| **15 Min** | > 4,18 | > 4,18 | > 4,18 | > 4,18 | > 4,18 |
| **30 Min** | > 4,16 | > 4,16 | > 4,16 | > 4,16 | > 4,16 |
| **60 Min** | > 4,27 | > 4,27 | > 4,27 | > 4,27 | > 4,27 |
| | | | | | |

| **Beispiel 8: Dimethyldiethanolammonium-di-laurinsäureester Methosulfat** | | | | | |
|---|---|---|---|---|---|
| **Staphyloccocus aureus ATCC 6538** | | | | | |
| **Keimzahl 4,23 x 10**^{**9**}**/ ml** | | | | | |
| **AWK [mg/l]** | | | **100** | **200** | **400** |
| **5 Min** | | | < 1,39 | 1,98 | 2,34 |
| **15 Min** | | | 3,24 | 3,40 | 4,64 |
| **30 Min** | | | 4,28 | 5,29 | > 5,51 |
| **60 Min** | | | 4,23 | > 5,60 | > 5,60 |

| **Pseudomonas aruginosa ATCC 15442** | | | | | |
|---|---|---|---|---|---|
| **Keimzahl 8,5 x 10**^{**9**} **/ ml** | | | | | |
| **AWK [mg/l]** | | | **100** | **200** | **400** |
| **5 Min** | | | < 1,88 | < 1,88 | < 1,88 |
| **15 Min** | | | < 1,93 | < 1,93 | < 1,93 |
| **30 Min** | | | < 1,80 | < 1,80 | 3,05 |
| **60 Min** | | | < 1,90 | 3,05 | 3,77 |
| | | | | | |

| **Beispiel 9: Methyltriethanolammonium-tri-caprinsäureester Methosulfat** | | | | | |
|---|---|---|---|---|---|
| **Staphyloccocus aureus ATCC 6538** | | | | | |
| **Keimzahl 4,23 x 10**^{**9**} **/ ml** | | | | | |
| **AWK [mg/l]** | | | **50** | **100** | **200** |
| **5 Min** | | | < 1,39 | < 1,39 | < 1,39 |
| **15 Min** | | | < 1,59 | < 1,59 | < 1,59 |
| **30 Min** | | | < 1,51 | < 1,51 | 1,83 |
| **60 Min** | | | < 1,60 | 2,09 | 2,97 |

| **Pseudomonas aruginosa ATCC 15442** | | | | | |
|---|---|---|---|---|---|
| **Keimzahl 8,5 x 10**^{**9**} **/ ml** | | | | | |
| **AWK [mg/l]** 3 | | | **100** | **200** | **400** |
| **5 Min** | | | < 1,88 | < 1,88 | < 1,88 |
| **15 Min** | | | < 1,93 | < 1,93 | < 1,93 |
| **30 Min** | | | < 1,80 | < 1,80 | 3,27 |
| **60 Min** | | | < 1,90 | 2,94 | 3,72 |
| | | | | | |

| **Beispiel 10: Dimethyldiethanolammonium-di-caprinsäureester Methosulfat** | | | | | |
|---|---|---|---|---|---|
| **Staphyloccocus aureus ATCC 6538** | | | | | |
| **Keimzahl 5,95 x 10**^{**9**} **/ ml** | | | | | |
| **AWK [mg/l]** | | | **100** | **200** | **400** |
| **5 Min** | | | > 5,56 | > 5,56 | > 5,56 |
| **15 Min** | | | > 5,55 | > 5,55 | > 5,55 |
| **30 Min** | | | > 5,72 | > 5,72 | > 5,72 |
| **60 Min** | | | > 5,69 | > 5,69 | > 5,69 |

| **Pseudomonas aruginosa ATCC 15442** | | | | | |
|---|---|---|---|---|---|
| **Keimzahl 9,45 x 10**^{**9**} **/ ml** | | | | | |
| **AWK [mg/l]** | | | **100** | **200** | **400** |
| **5 Min** | | | 3,11 | 3,54 | 4,31 |
| **15 Min** | | | 3,48 | 4,51 | 5,04 |
| 30 Min | | | 3,95 | 5,50 | 5,64 |
| **60 Min** | | | 4,84 | 5,44 | > 5,79 |

| **Candida albicans ATCC 10231** | | | | | |
|---|---|---|---|---|---|
| **Keimzahl 1,03 x 10**^{**9**} **/ ml** | | | | | |
| **AWK [mg/l]** | | | **50** | **100** | |
| **5 Min** | | | 2,87 | 4,27 | |
| **15 Min** | | | > 4,84 | > 4,84 | |
| **30 Min** | | | > 4,89 | > 4,89 | |
| **60 Min** | | | > 4,80 | > 4,80 | |

### B. Mikrobizide Wirksamkeit im Hinblick auf weitere Bakterien und Pilze

Analog A) wurde Methyltriethanolammonium-di-octansäureester Methosulfat gegenüber den

| | |
|---|---|
| Bakterien | - Salmonella Enteritidis |
| | - Escherichia coli |
| | - Enterococcus faecium |
| und den | |
| Pilzen | - Microsporum gypseum |
| | - Trichophyton mentagrophytes |

getestet. In Tabelle 2 sind die logarithmischen Reduktionsraten dieses Beispiels 11 wiedergegeben.

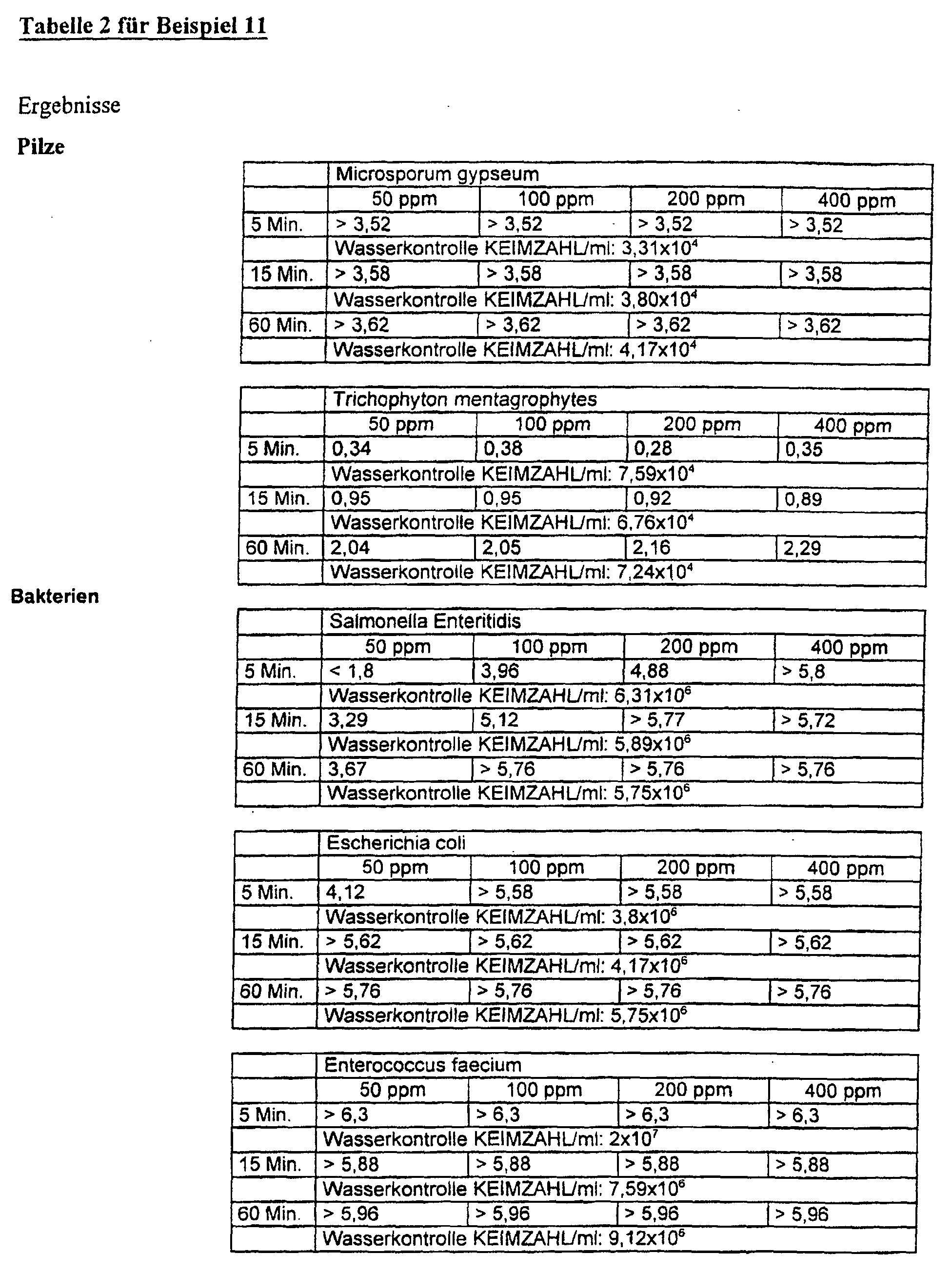

Die gute Wirksamkeit gegen Bakterien wurde durch die vorliegenden Prüfergebnisse bestätigt. Es zeigte sich insbesondere eine gute Wirksamkeit gegen Gram-positive Bakterien.

Der Pilz *Microsporum gypseum* wird bereits bei der kürzesten Einwirkzeit von einer Konzentration von 50 mg/l praktisch vollständig abgetötet. Gegen den Dermatophyten *Trichophyton mentagrophytes* wird zumindest nach einer langen Einwirkzeit (60 Minuten) eine beginnende Wirksamkeit mit einer Abtötung um 2 Zehnerpotenzen beobachtet.

## Patentansprüche

1. Verwendung von quaternären Carbonsäurealkanolaminestersalzen der allgemeinen Formel (I) 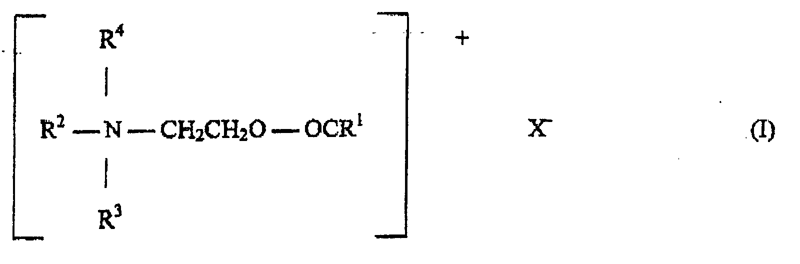 in der
R¹ CO für einen Acylrest mit 2 bis 18 C-Atomen
R² für einen Rest der Formel CH₂-CH₂-O-OCR¹,
R³ für einen Alkylrest mit 1 bis 16 C-Atomen oder für einen Rest der Formel CH₂-CH₂-O-R⁵,
R⁴ für einen Alkylrest mit 1 bis 4 C-Atomen,
R⁵ für Wasserstoff und/oder R¹CO und
X⁻ für ein Anion steht
als mikrobizide Wirkstoffe.

2. Verwendung nach Anspruch 1, **dadurch gekennzeichnet, daß** quaternäre Carbonsäurealkanolaminestersalze der Formel (I) verwendet werden, in der R¹CO für einen aliphatischen gesättigten Acylrest mit 8 bis 12 C-Atomen steht.

3. Verwendung nach Anspruch 1 oder 2, **dadurch gekennzeichnet, daß** quaternäre Carbonsäurealkanolaminestersalze der Formel (I) verwendet werden, in der R² für einen Rest der Formel CH₂CH₂OOCR¹und R³ für eine Methylgruppe steht.

4. Verwendung nach einem der Ansprüche 1 bis 2, **dadurch gekennzeichnet, daß** quaternäre Carbonsäurealkanolaminestersalze der Formel (I) verwendet werden, in der R² für einen Rest der Formel CH₂CH₂OOCR¹ und R³ für einen Rest der Formal CH₂CH₂OR⁵ steht.

5. Verwendung nach einem der Ansprüche 1 bis 4, **dadurch gekennzeichnet, daß** quaternäre Carbonsäurealkanolaminestersalze der Formel (I) verwendet werden, in der R⁴ für eine Methylgruppe steht.

6. Verwendung nach einem der Ansprüche 1 bis 5, **dadurch gekennzeichnet, daß** X für ein Anion steht ausgewählt aus der von Methosulfat, Ethosulfat, Formiat, Acetat, Tartrat, Dicarboxylat, Citrat, Halogenid, Sulfat, Phosphat und Nitrat gebildeten Gruppe.

7. Verwendung nach einem der Ansprüche 1 bis 6, **dadurch gekennzeichnet, daß** die quaternären Carbonsäurealkanolaminestersalze der Formel (I) als mikrobizide Wirkstoffe in Wasch- und Reinigungsmitteln, Desinfektionsmitteln, Konservierungsmitteln, Kosmetika, Weichspülern, Kühlschmieremulsionen und in Anstrichmitteln verwendet werden.

8. Verwendung nach einem der Ansprüche 1 bis 7, **dadurch gekennzeichnet, daß** die quaternäre Carbonsäurealkanolaminestersalze der Formel (I) in Mengen von 5 - 5000 mg/l - berechnet als Aktivsubstanz und bezogen auf das Mittel - verwendet werden.

9. Wasch- und Reinigungsmittel, **dadurch gekennzeichnet, daß** sie als mikrobizide Wirkstoffe quaternäre Carbonsäurealkanolaminestersalze der Formel (I) enthalten.

10. Konservierungsmittel, **dadurch gekennzeichnet, daß** sie als mikrobizide Wirkstoffe quaternäre Carbonsäurealkanolaminestersalze der Formel (I) enthalten.

11. Desinfektionsmittel, **dadurch gekennzeichnet, daß** sie als mikrobizide Wirkstoffe quaternäre Carbonsäurealkanolaminestersalze der Formel (I) enthalten.

12. Kühlschmieremulsionen, **dadurch gekennzeichnet, daß** sie als mikrobizide Wirkstoffe quaternäre Carbonsäurealkanolaminestersalze der Formel (I) enthalten.

13. Kosmetika, **dadurch gekennzeichnet, daß** sie als mikrobizide Wirkstoffe quaternäre Carbonsäurealkanolaminestersalze der Formel (I) enthalten.

## Claims

1. The use of quaternary carboxylic acid alkanolamine ester salts corresponding to general formula (I): 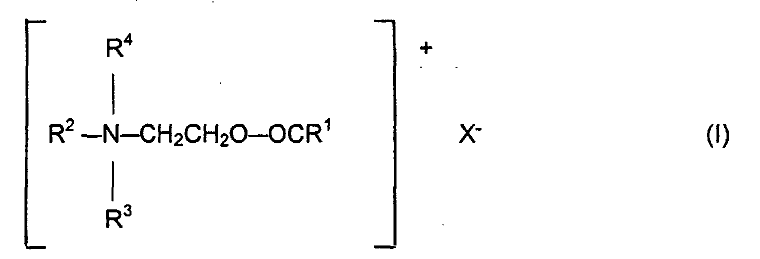 in which
R¹ CO is an acyl group containing 2 to 18 carbon atoms,
R² is a group with the formula CH₂-CH₂-O-OCR¹,
R³ is an alkyl group containing 1 to 16 carbon atoms or a group with the formula CH₂-CH₂-O-R⁵,
R⁴ is an alkyl group containing 1 to 4 carbon atoms,
R⁵ is hydrogen and/or R¹CO and
X- is an anion,
as microbicidal agents.

2. The use claimed in claim 1, **characterized in that** quaternary carboxylic acid alkanolamine ester salts corresponding to formula (I), in which R¹CO is an aliphatic saturated acyl group containing 8 to 12 carbon atoms, are used.

3. The use claimed in claim 1 or 2, **characterized in that** quaternary carboxylic acid alkanolamine ester salts corresponding to formula (I), in which R² is a group with the formula CH₂CH₂OOCR¹ and R³ is a methyl group, are used.

4. The use claimed in any of claims 1 to 2, **characterized in that** quaternary carboxylic acid alkanolamine ester salts corresponding to formula (I), in which R² is a group with the formula CH₂CH₂OOCR¹ and R³ is a group with the formula CH₂CH₂OR⁵, are used.

5. The use claimed in any of claims 1 to 4, **characterized in that** quaternary carboxylic acid alkanolamine ester salts corresponding to formula (I), in which R⁴ is a methyl group, are used.

6. The use claimed in any of claims 1 to 5, **characterized in that** X is an anion selected from the group consisting of methosulfate, ethosulfate, formate, acetate, tartrate, dicarboxylate, citrate, halide, sulfate, phosphate and nitrate.

7. The use claimed in any of claims 1 to 6, **characterized in that** the quaternary carboxylic acid alkanolamine ester salts corresponding to formula (I) are used as microbicidal agents in detergents, disinfectants, preservatives, cosmetics, fabric softeners, cooling lubricant emulsions and in coating compositions.

8. The use claimed in any of claims 1 to 7, **characterized in that** the quaternary carboxylic acid alkanolamine ester salts corresponding to formula (I) are used in quantities of 5 to 5000 mg/l, expressed as active substance and based on the particular preparation.

9. Detergents, **characterized in that** they contain quaternary carboxylic acid alkanolamine ester salts corresponding to formula (I) as microbicidal agents.

10. Preservatives, **characterized in that** they contain quaternary carboxylic acid alkanolamine ester salts corresponding to formula (I) as microbicidal agents.

11. Disinfectants, **characterized in that** they contain quaternary carboxylic acid alkanolamine ester salts corresponding to formula (I) as microbicidal agents.

12. Cooling lubricant emulsions, **characterized in that** they contain quaternary carboxylic acid alkanolamine ester salts corresponding to formula (I) as microbicidal agents.

13. Cosmetics, **characterized in that** they contain quaternary carboxylic acid alkanolamine ester salts corresponding to formula (I) as microbicidal agents.

## Revendications

1. Utilisation de sels d'ester d'alkanolamine et d'acide carboxylique quaternaires de formule générale (I) 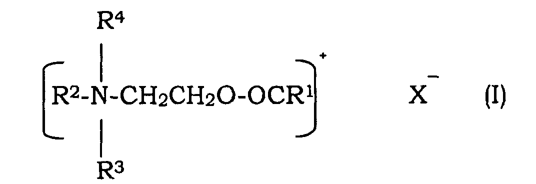 dans laquelle
R¹CO représente un reste acyle ayant de 2 à 18 atomes de carbone,
R² représente un reste de formule CH₂-CH₂-O-OCR¹,
R³ représente un reste alkyle ayant de 1 à 16 atomes de carbone ou un reste de formule CH₂-CH₂-OR⁵,
R⁴ représente un reste allyle ayant de 1 à 4 atomes de carbone,
R⁵ représente de l'hydrogène et/ou R¹CO et X⁻ représente un anion,
en tant que principes actifs microbicides.

2. Utilisation de sels d'ester selon la revendication 1,
**caractérisée en ce qu'**
on utilise de sels d'ester d'alkanolamine d'acide carboxylique quaternaires de formule (I) dans laquelle R¹CO représente un reste acyle aliphatique saturé, ayant de 8 à 12 atomes de carbone.

3. Utilisation de sels d'ester selon la revendication 1 ou la revendication 2,
**caractérisée en ce qu'**
on utilise de sels d'ester d'alkanolamine d'acide carboxylique quaternaires, de formule (I) dans laquelle R² représente un reste de formule CH₂CH₂OOCR¹ et R³ un groupe méthyle.

4. Utilisation de sels d'ester selon l'une des revendications 1 à 2,
**caractérisée en ce qu'**
on utilise de sels d'ester d'alkanolamine d'acide carboxylique quaternaires de formule (I) dans laquelle R² représente un reste de formule CH₂CH₂OOCR¹ et R³ un reste de formule CH₂CH₂OR⁵.

5. Utilisation de sels d'ester selon l'une des revendications 1 à 4,
**caractérisée en ce qu'**
on utilise des sels d'ester d'alkanolamine d'acide carboxylique quaternaires de formule (I) dans laquelle R⁴ représente un groupe méthyle.

6. Utilisation de sels d'ester selon l'une des revendications 1 à 5,
**caractérisée en ce que**
X représente un anion choisi dans le groupe formé des méthosulfate, éthosulfate, formiate, acétate, tartrate dicarboxylate, citrate, halogénure, sulfate, phosphate, et des nitrates.

7. Utilisation de sels d'ester selon l'une des revendications 1 à 6,
**caractérisée en ce qu'**
on utilise les sels d'ester d'alkanolamine d'acide carboxylique quaternaires de formule (I) comme principes actifs microbicides dans des produits de lavage et de nettoyage, des agents désinfectants, des agents conservateurs, des cosmétiques, des agents assouplissants, des émulsions d'agent réfrigérant de lubrifiant et dans des peintures.

8. Utilisation de sels d'ester selon l'une des revendications 1 à 7,
**caractérisée en ce qu'**
on utilise les sels d'esters d'alkanolamine d'acide carboxylique quaternaires de formule (I) en quantité allant de 5 à 5000 mg/l calculé comme substance active et rapporté à la composition.

9. Produits de lavage et de nettoyage,
**caractérisés en ce qu'**
ils renferment comme principes actifs microbicides des sels d'ester d'alkanolamine d'acide carboxylique quaternaires de formule (I).

10. Agents conservateurs,
**caractérisés en ce qu'**
ils renferment comme principes actifs microbicides, des sels d'ester d'alkanolamine d'acide carboxylique quaternaires de formule (I).

11. Agents désinfectants,
**caractérisés en ce qu'**
ils renferment comme principes actifs microbicides des sels d'ester d'alkanolamine d'acide carboxylique quaternaires de formule (I).

12. Emulsions d'agent réfrigérant de lubrifiant,
**caractérisées en ce qu'**
elles renferment comme principes actifs microbicides, des sels d'ester d'alkanolamine d'acide carboxylique quaternaires, de formule (I).

13. Cosmétiques,
**caractérisés en ce qu'**
ils renferment comme principes actifs microbicides des sels d'ester d'alkanolamine d'acide carboxylique quaternaires de formule (I).
